(19) **Europäisches Patentamt / European Patent Office / Office européen des brevets**

(11) **EP 4 489 591 B1**

(12) # EUROPEAN PATENT SPECIFICATION

(45) Date of publication and mention
of the grant of the patent:
**14.01.2026 Bulletin 2026/03**

(21) Application number: **23715936.3**

(22) Date of filing: **10.03.2023**

(51) International Patent Classification (IPC):
*A23L 11/00* (2025.01)    *A23P 10/28* (2016.01)
*A23P 10/30* (2016.01)    *A61J 3/10* (2006.01)
*A61K 9/20* (2006.01)    *A23L 19/00* (2016.01)
*A23L 25/00* (2016.01)    *A23L 29/25* (2016.01)

(52) Cooperative Patent Classification (CPC):
**A23L 29/25; A23L 11/00; A23L 19/01; A23L 25/20;
A23P 10/28; A23P 10/30; A61K 9/205;
A61K 9/2068**

(86) International application number:
**PCT/IB2023/052293**

(87) International publication number:
**WO 2023/170644 (14.09.2023 Gazette 2023/37)**

(54) **NEW NATURAL SYSTEMS WITH TECHNOLOGICAL LUBRICANT ACTION**

NEUE NATÜRLICHE SYSTEME MIT TECHNOLOGISCHER SCHMIERSTOFFWIRKUNG

NOUVEAUX SYSTÈMES NATURELS À ACTION LUBRIFIANTE TECHNOLOGIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **10.03.2022 IT 202200004637**

(43) Date of publication of application:
**15.01.2025 Bulletin 2025/03**

(73) Proprietor: **Aboca S.p.A. Società Agricola
52037 Sansepolcro (AR) (IT)**

(72) Inventors:
• **MERCATI, Valentina
52037 Sansepolcro (ar) (IT)**
• **BULGARI, Ambra
52037 Sansepolcro AR (IT)**

• **ZAMBALDI, Ilaria
52037 Sansepolcro AR (IT)**
• **GIOVAGNONI, Emiliano
52037 Sansepolcro (ar) (IT)**

(74) Representative: **Predazzi, Valentina et al
Società Italiana Brevetti S.p.A.
Piazza di Pietra, 39
00186 Roma (IT)**

(56) References cited:
**CN-A- 106 072 373    CN-A- 106 173 822
CN-A- 110 074 242    JP-A- 2007 320 856
JP-A- 2021 061 825    KR-A- 20200 009 218
US-A1- 2013 296 445    US-A1- 2020 170 936
US-B1- 6 207 189**

## EP 4 489 591 B1

**Description**

## FIELD OF THE INVENTION

The present invention relates to systems with emerging properties, in particular systems suitable for being used as lubricating agents, for example for the manufacture of tablets and capsules, and as technological adjuvants for the functioning of the processing machines for the preparation of said materials, and provides new natural systems with lubricating action (suitable for use as lubricating agents) of 100% natural origin, compositions comprising them, use and processes for preparation of said agents. In particular, the present invention provides new natural systems with a lubricating activity (suitable for use as lubricating agents) composed 100% by products of natural origin (not of chemical synthesis), consisting of powders of fruits comprising seeds or of seeds in which said fruits or seeds are characterized by the presence of fatty acids, optionally in combination with natural rubbers, in order to replace the commonly used powder lubricants of chemical origin.

## STATE OF THE ART

Lubricating agents are substances capable of preventing the adhesion to the surface of the punch matrix of the powders to be compressed, reducing friction and facilitating the expulsion of the tablets from the matrix. These raw materials can be used directly in the mixture and/or externally to the formula (technological adjuvants) to obtain compliant tablets or capsules.

The lubricating action of an agent and its effectiveness (whether when used as an additive or as a technological adjuvant or both) can be assessed, for example, by monitoring through the ejection force of a tablet detected throughout the entire compression process.

Furthermore, the fundamental properties of the powders (particle size and distribution or granulometry and specific surface area) and the derived properties (bulk density and flowability) play an important role in obtaining compliant solid pharmaceutical forms.

In conventional production process schemes used for the manufacture of tablets and capsules of products of vegetable origin, the addition of stearates of chemical origin is foreseen to make the mixtures lubricated. Alternatively the use of stearates is foreseen as technological adjuvants for the functioning of the process machines and in this case it is added externally leaving traces of the adjuvant on the surface of the product.

Magnesium stearate is one of the most used lubricating agents worldwide and is obtained from a reaction in an aqueous environment between magnesium chloride or magnesium oxides and stearic acid at high temperatures. Prolonged mixing times would lead to the formation of lamellar layers capable of covering the active ingredients, effectively sequestering them, and delaying their bioavailability.

There is therefore the need to provide mixtures which allow to avoid the use of stearates of chemical origin both as ingredients of the formula as well as technological adjuvants in the conventional production processes used for the manufacture of capsules and tablets of products of natural origin, allowing to obtain 100% natural products.

Document JP2007320856 describes a lubricant that uses the properties of fats and oils present in vegetable raw material, in particular soybeans, document US2020/170936 describes a rice husk-based lubricant, document KR2020/0009128 describes a lubricant based on of cocoa powder. The type and quantity of vegetable fats which must be present in said lubricating agents in order to provide a technical effect comparable to that of magnesium stearate are not reported in the literature. Although some lubricating agents for tablets consisting of natural substances, such as for example derivatives of seeds and/or fats of some plants, begin to be described in the state of the art, lubricants consisting of 100% natural ingredients technically equivalent to magnesium stearate do not appear to be available.

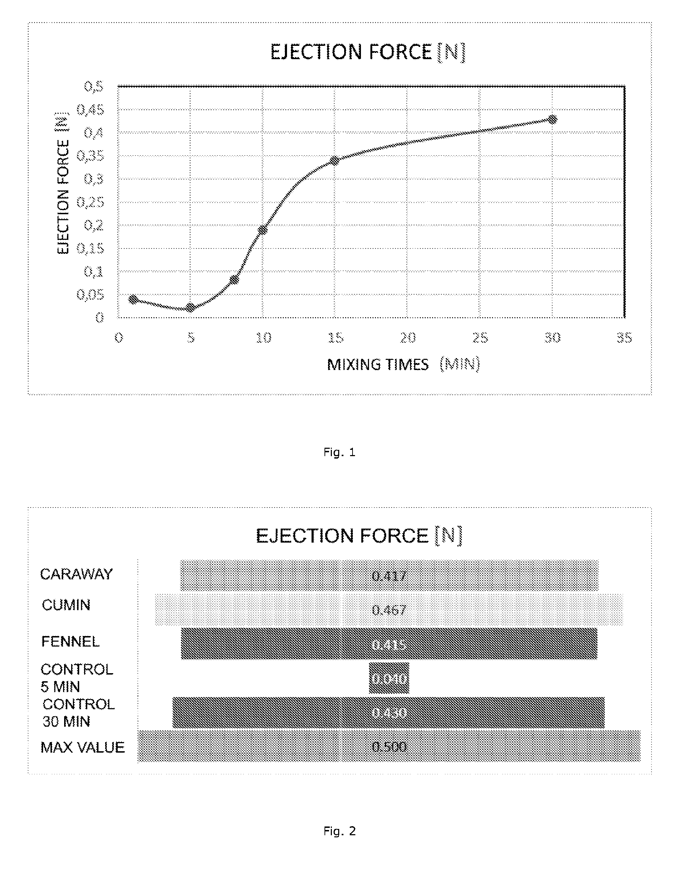

DETAILED DESCRIPTION OF Figure 1. Target curve of the ejection force [N] for the definition of a range of values attributable to ejection forces which guarantee the detachment of the tablet from the punch (experiment 4 example section). Given a mixture of standard powders (also called standard mixture), consisting of microcrystalline cellulose and calcium carbonate or limestone powder in a weight ratio of 4:1, performance tests were carried out with compositions comprising said mixture and 1% of magnesium stearate (w/w of the composition) at different mixing times, and the ejection force of the tablets was evaluated. The mixing times were related to the ejection forces obtained to construct a target curve. The measurement carried out in figure 1 allows to establish the range of expulsion forces exerted by the magnesium stearate to be used as a reference for comparative tests. In particular, two parameters have been identified: the mixing time for which the lowest ejection force value of the mixture is obtained, equal to 5', and the mixing time for which the highest ejection force value of the mixture is obtained, equal to 30'.

The range of ejection forces considered valid on the basis of the quality of the tablets obtained is from 0.05 to 0.50 N.

**Figure 2. Compared ejection forces of powders of different fruits comprising seeds (experiment 5 section examples).**

Compression tests were carried out in which compositions consisting of a standard mixture according to the present description with the addition of powders of fruits comprising seeds of Caraway, Fennel and Cumin taken individually in a percentage by weight with respect to the total weight of the composition, which contained a quantity of fatty acids approximately equivalent to that present in 1% magnesium stearate in the same formula calculated according to the desired percentage of fatty acids on the basis of table 1 in the description were used. In particular, the ejection force was evaluated in compositions comprising powders of fruits consisting of seeds (achenes) of fennel, caraway and cumin, and the standard mixture as described above (microcrystalline cellulose and calcium carbonate in a weight ratio of 4: 1). The results obtained with compositions (standard mixture and powders of fruits comprising seeds) in which the percentage by weight of powder of fruits comprising fennel seeds with respect to the total weight of the composition was 4.21%, in which the percentage by weight of powder of fruits comprising caraway seeds to the total weight of the composition was 3.75%, wherein the percentage by weight of powder of fruits comprising caraway seeds to the total weight of the composition was 3.91%, using the same mixing time optimal found for magnesium stearate, equal to 5 minutes.

The tablet ejection force values were then analyzed and compared to the parameters obtained from the compression of a standard mixture (consisting of microcrystalline cellulose and calcium carbonate or limestone powder in a weight ratio of 4:1) further containing 1% by weight, with respect to the total weight of the formulation (standard mixture + stearate) of magnesium stearate and to the maximum experimental expulsion force value on other products, the generally accepted value of which corresponds to 0.5 N. The control is represented by the compositions with mixing times of 5 and 30 minutes described for figure 1.

All compression tests were performed with the same operating parameters. Although in all tests the ejection force measured is less than 0.5 N, the values obtained tend to the upper limit (i.e. 0.5 N) set in the study on the basis of the values measured for magnesium stearate in figure 1 .

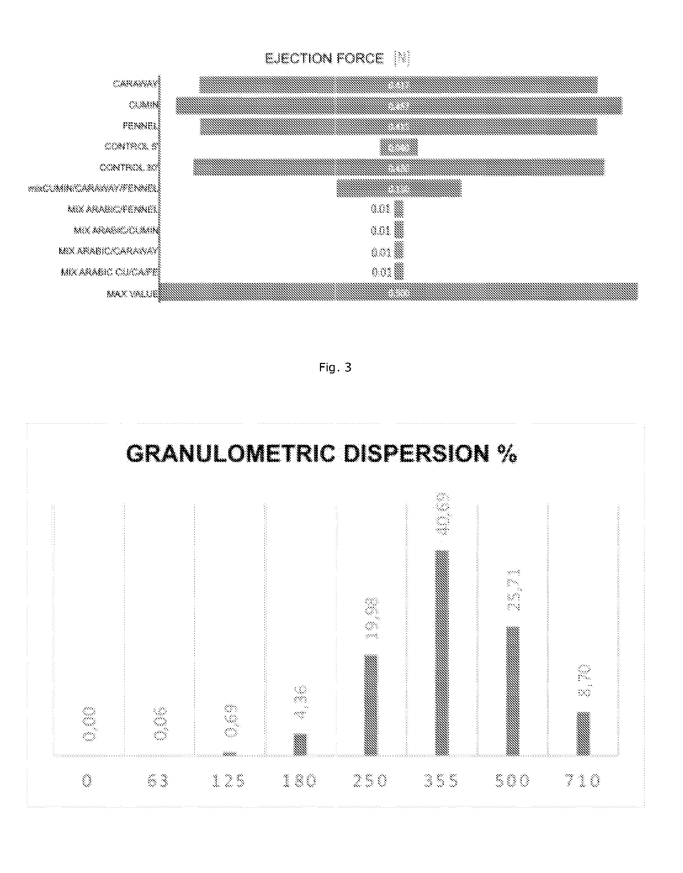

**Figure 3. Ejection forces of mixtures containing a mixture of powders of seed and/or fruit and gum arabic (aka acacia gum) (experiment 6 section examples).** Systems consisting of powdered gum arabic and powders of fruits comprising seeds or od fennel, caraway, cumin or a mixture of said powders of fruits comprising seeds, in a 7:1 ratio, were premixed and the resulting mixture was inserted into a standard mixture as described above at a concentration of 32.34% (4.21% of powders of fruits comprising fennel, caraway and cumin seeds in a ratio of 1:1:1, and 28.13% of gum arabic) by weight to the total weight of the resulting composition (standard mix + mixture of powders of fruits comprising seeds and gum arabic). The values of ejection force obtained compared with the control (standard mixture and 1% by weight of magnesium stearate) and with the compositions described above containing the single fruits comprising seeds in powder (fennel at 4.21%, caraway 3.75%, cumin 3.91%) are reported below. Under the same operating conditions, the compositions consisting of standard mixture, arabic gum and powders of several fruits comprising seeds (fennel, caraway and cumin) contribute to producing lower ejection force values compared to the compositions consisting of standard mixture and powder of a single type of fruit comprising seeds (fennel, caraway or cumin) and also with respect to the composition consisting of standard mixture and magnesium stearate.

The exact values obtained with the experiment depicted in figure 3 are shown in table 4, experiment 6.

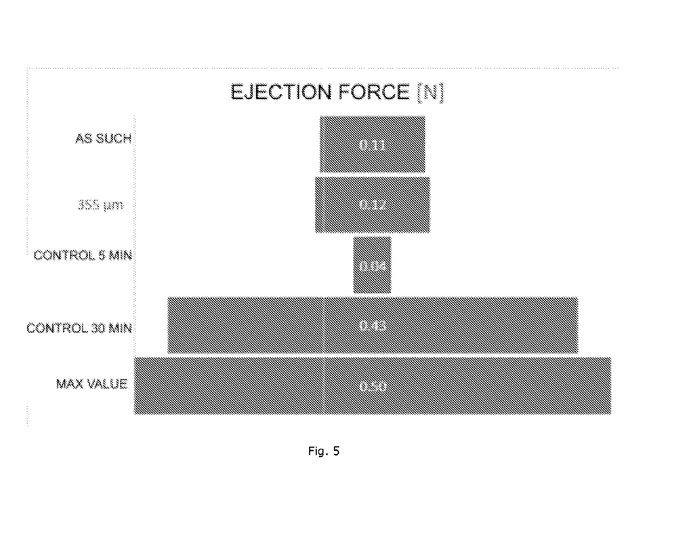

**Figure 4. Distribution curve obtained by sieving with the sieve method (experiment 7 examples section).** A mixture of gum arabic and powders of fruits comprising fennel seeds in a 5:1 ratio was granulated with water and sifted through a vibrating sieve. The granulate obtained was subjected to particle size analysis, the results of which are shown in figure 4. The characterization of the properties of the granulate is shown in table **8.Figure 5. Ejection forces in comparison (experiment 7 examples section).**

The ejection force values obtained, compared to the values obtained for the control as defined above are reported. The "gum-fennel" pre-granulated system shows different ratios with respect to those of the compositions of Figure 3, said pre-granulate consisting of 2% by weight of powder of fruit comprising fennel seeds, and 10% by weight of gum arabic, for a total of 12% by weight in a composition comprising said granulate and standard mixture. The figure demonstrates that this system allows to obtain tablets without the addition of magnesium stearate either in the formula or externally. Furthermore, the quantity of the processed system added to the blend is considerably lower than that used in the compositions of Figure 3, thus demonstrating that quantities of gum arabic and powder of fruits comprising seeds, even much lower than those tested in Figure 3, are also sufficient. The ejection force of the pre-granulate described in Figure 4 was also compared with respect to the same quantity of the 355 $\mu$m particle size fraction thereof.

The pre-granulate and its fraction have comparable lubricating properties.

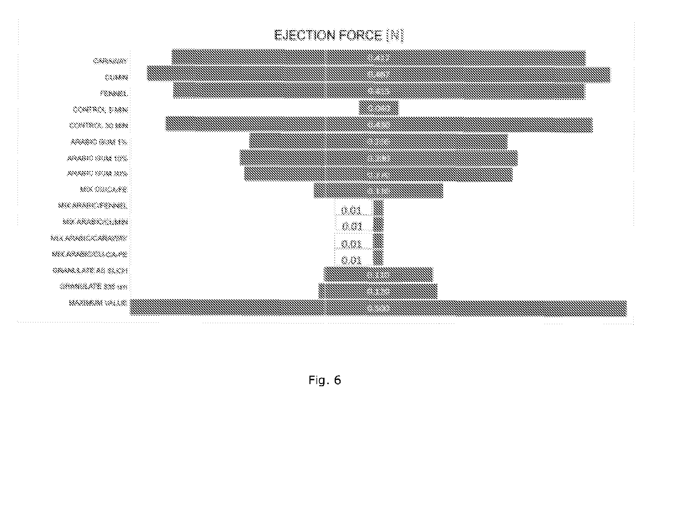

**Figure 6. Global graph of ejection forces (experiment 6 examples section, table 5).** All the ejection force values of the compositions described in table 5 are reported. The figure shows the completely unexpected synergistic effect both in compositions in which the natural lubricating system consists of a mixture of powders of different fruits comprising seeds, and, even more effectively, in compositions whose natural lubricating system consists of gum arabic and powder of one or more fruits comprising seeds.

## GLOSSARY

In the present description, the term "I.C. Carr's index" refers to a dimensionless physical quantity that characterizes the compressibility of a powder or of a granular material.

In the present description, the term "I.H. Hausner index" refers to the ratio between the density after compression and the initial density of the powder. This parameter is useful for evaluating the flowability of a powder or of a mixture of powders.

The term "Bulk density" in the present description refers to the density of the powder considering inter- and intra-particle spaces.

The term "Tapped density" in the present description refers to the density value after packing. The terms "Dv10", "Dv50" and "Dv90" and "SPAN" refer to:

- Dv10: 10% of the volume of distribution of the particle diameter values is below the indicated value.
- Dv50: 50% of the volume of distribution of the particle diameter values is below the indicated value. Mean volume of distribution value.
- Dv90: 90% of the volume of distribution of the particle diameter values is below the indicated value.

- SPAN: is obtained using the following formula $SPAN = \frac{Dv90 - Dv10}{Dv50}$.

A value corresponding to 1 corresponds to a Gaussian distribution.

The term "granulation" refers to a technological transformation of a substance or of a material into granules, for the purpose of definitive use or further processing.

The term "compression" refers to a production process aimed at obtaining tablets by mechanical means.

The term "ejection force" refers to the experimental parameter that allows to measure the phenomenon of adhesion of the tablet to the punch, measured in [N]. For the purposes of this description, the maximum value of ejection force considered acceptable is equal to 0.5 N, beyond which technological problems related to friction such as the adhesion of the tablet on the punches of the machine, sticking, and descaling are observed. The value of the ejection force is an index of the effectiveness of the lubricating system used, in particular a good lubricant allows to lower the value of the ejection force down to values below 0.1 N. The term "system with emerging properties" refers, in the present description, to a system, in this case a mixture or a composition, which exhibits an emergent behaviour, i.e., the agents constituting the mixture, operating in a given environment, give rise to more complex behaviours as they are in the form of a system. The properties themselves are not easily predictable and represent a subsequent level of evolution of the system. Complex emergent behaviours are not properties of single entities and it is in fact necessary to implement the entire system in order to obtain the observed emergent behaviour.

In the present invention 100% natural means that what is defined as 100% natural does not contain any components obtained from chemical synthesis but only components of natural origin, in particular, products from plants. The term "active ingredient" in the present description refers to a substance endowed with a specific biological activity, including all substances which perform a specific activity in the formulation, for example with a therapeutic or preventive effect.

In the present description, a standard mixture of powders, also standard mixture, is a mixture consisting of microcrystalline cellulose and calcium carbonate or limestone powder in a weight ratio between them of 4:1, and a lubricant agent or system with variable concentration depending on the type of lubricant.

In the present description, the expression "physiologically active system" refers to a physiologically active system whose constituents interact with each other both structurally and functionally in order to determine emergent properties. The system thus described is physiologically active as it is able to interact with the living body by acting on multilevel mechanisms without artificially modifying single and specific functions. In the present description, the term "natural raw material in powder" refers to a natural raw material (such as seeds, fruits comprising seeds, fruits) dried and pulverized by means of a grinding process.

In the present description, the term "relative humidity" refers to the ratio of the density of the vapor contained in a substance and the density of the saturated vapor at the temperature of the substance. It is an index of the amount of water vapor contained in a substance.

In the present description, the term "formulation preparation" refers to a mixture of active ingredients and optionally excipients, colourings, flavourings, and carriers, intended for the preparation of a product in the form of a capsule or tablet. The formulation preparation can be easily identified by the skilled person, and is any formulation in which magnesium stearate is normally usable as a lubricating agent.

By fruits comprising seeds the whole fruit, therefore comprising the seeds contained therein is intended.

## SUMMARY OF THE INVENTION

The invention is defined by the claims.

The authors of the present invention, in order to be able to create products made entirely of natural substances, and therefore not of chemical synthesis, have tried to identify natural systems with a lubricating technological activity, hence suitable for being used as lubricating agents in the manufacturing processes of tablets and capsules and in machinery for such manufacturing, in which such systems present a different technological action, for example in terms of effectiveness, from that exerted by the single components of such systems. In order to obtain lubricating agents consisting of 100% natural substances and with an effectiveness comparable to that of magnesium stearate, the inventors have verified the lubricating properties, in the manufacture of tablets or capsules, of powders of different fruits comprising seeds, or of powders of seeds, comprising fatty acids with chain lengths similar to those present in magnesium stearate and evaluated the lubricating activity of these powders compared to magnesium stearate. The grinding of fruits comprising seeds or of seeds allows these materials to be transformed into powder and to be blended effectively into 100% natural product formulas. In addition, the grinding process allows the exposure of the lipid components, which interact effectively with powders or granulates of the active substances of the product, thereby allowing an effective lubrication.

This effective contact allows the mixture obtained to be transformed into capsules and/or tablets, guaranteeing the functionality of the technologies used for these transformations, thereby avoiding the use of stearates of chemical origin both as ingredients of the formula and as technological adjuvants in the conventional production processes used for the manufacturing of capsules and tablets of products of natural origin, hence allowing to obtain 100% natural products.

Furthermore, the authors of the present invention have surprisingly discovered that natural gums, such as gum arabic, even when used as the sole lubricating agent or as a component of a lubricating agent, have a lubricating effect in the preparation of tablets and capsules.

As shown in figure 2, the control at the optimal mixing time derived from the curve of figure 1 shows an ejection force value lower than 0.1 N thanks to the effectiveness of magnesium stearate in the lubricating action, while the powders of fruits comprising seeds show, at the same mixing time considered optimal for control at t min, ejection force values close to the one considered as the maximum acceptability limit of ejection force values for the purposes of the present invention. While all individual powders of fruits comprising seeds or of seeds tested provided an "acceptable" lubricating activity, it has not been possible to obtain the excellent ejection force values observed for magnesium stearate.

Surprisingly, the authors found that suitable blends of powders of fruits comprising seeds or seed powders give a synergistic effect and provide a system with emerging properties in which the lubricating activity is unexpectedly significantly better than that of the single powders, and, that this effectiveness is even greater following the mixing of one or more powders of fruits comprising seeds, with vegetable gum/powder, which provide 100% natural systems with a lubricating action (suitable for use as lubricating agents), that is even better than the one of magnesium stearate. Furthermore, the authors of the present invention have surprisingly discovered that the same vegetable gums are capable of providing, *per se,* an effective lubricating activity in the manufacture of capsules and tablets. In other words, the authors of the invention have surprisingly discovered that the association of vegetable gums and powder of particular fruits comprising seeds or of seeds, or the association of different powders of fruit comprising seeds or of seeds, optionally in combination with vegetable gums (herein also referred to as natural gums) provides an system with emergent properties with excellent lubricating properties comparable to, if not better than, those exhibited by one of the most common and effective synthetic chemical lubricants for tablet and capsule manufacturing, i.e. magnesium stearate in its optimal conditions of use, i.e. mixing time 5 minutes (see figure 6).

The system of the invention, presented here in the form of a mixture or composition, thus provides an excellent lubricating agent suitable for the manufacture of 100% natural formulas.

The following are therefore the object of the invention:- a mixture consisting of one or more fruit comprising seeds or seed species in the form of powder and natural gum powder or of a mixture of at least three different species of seeds in powder, wherein the fruits comprising seeds or the seeds in powder from each of said species contain $C_{16}$-$C_{18}$ fatty acids in a percentage greater than 45% w/w with respect to the total fatty acids and a percentage of fatty acids of at least 21% w/w. wherein said species of fruits comprising seeds are cumin, caraway and fennel and, wherein said fruit comprising seeds or seed powders of cumin, caraway and fennel have a weight ratio from 1:1:1 to 1:1:3, preferably 1:1:1.

- a composition comprising the mixture of the present invention as sole lubricating agent;
- the use of the mixture object of the invention, as a lubricating agent in the preparation of tablets, capsules, solid compositions;
- a process for manufacturing capsules or tablets or solid compositions comprising the step of admixing the mixture of the invention with a suitable formulation preparation, wherein said mixture is the sole lubricating agent used in said process;

- a process for manufacturing capsules or tablets or solid compositions comprising the following steps:

   i) preparing one or more fruit comprising seeds or seed species in the form of powder and/or natural gum, wherein the fruits comprising seeds or the seeds in powder from each of said species contain $C_{16}$-$C_{18}$ fatty acids in a percentage higher than 45% w/w with respect to total fatty acids and a percentage of fatty acids of at least 21% w/w wherein said species are cumin, caraway and fennel and wherein said cumin, caraway and fennel have a weight ratio from 1:1:1 to 1:1:3, preferably 1:1:1;

   ii) mixing said one or more species of cumin, caraway and/or fennel fruit comprising seeds or seeds in the form of powder and/or said natural gum with a suitable formulation preparation for a time from 2 to 30 minutes, preferably from 5 to 15 minutes, even more preferably 5 minutes;

   iii) subjecting the product obtained in step ii) to compression or encapsulation wherein the components used in steps i) and ii) constitute (are) the sole lubricating agent used in said process

- capsules, tablets or solid compositions obtainable by said process .

Further advantages and/or embodiments of the present invention will be evident from the following detailed description.

DETAILED **DESCRIPTION**

The authors of the present invention have surprisingly discovered that the association of vegetable gums and powders of fruit comprising seeds or of seeds; or the association of different powders of fruits comprising seeds or of seeds, provides a system with emerging properties with excellent lubricants properties, comparable to those shown by one of the most common chemical synthesis lubricants for the manufacture of tablets and capsules, namely magnesium stearate, in its optimal conditions of use.

Therefore, an object of the present invention is represented by a mixture consisting of one or more species of fruit comprising seeds or seeds in powder form and natural gum in powder or by a mixture of at least three different species of fruit comprising seeds or seeds in powder form wherein the fruits comprising seeds or powdered seeds of each of said species contain $C_{16}$-$C_{18}$ fatty acids in a percentage greater than 45% w/w compared to the total fatty acids and a percentage of fatty acids of at least 21% w/w in which said species of fruits comprising seeds or powdered seeds are cumin, caraway and fennel and wherein said cumin, caraway and fennel have a weight ratio from 1:1:1 to 1:1:3, preferably 1:1:1.

As can be seen from table 1 in the Examples section of the present description, said $C_{16}$-$C_{18}$ fatty acids present in the seed powders are mainly oleic acid and linoleic acid. According to the present invention, said species are preferably characterized in that their dried fruits comprise orthodox seeds, i.e. seeds capable of resisting for long periods without germinating (years) while remaining viable.

The fennel fruit, or also Foeniculi fructus, consists of the schizocarps, also called "double achenes", and more often, of the single partial small fruits ("achenes"), 3-12 mm long and 2-4 mm broad, of yellowish-green to yellowish-brown. Fragments of the stylus are often found on the upper end of the stylopodium. Each "achene" has 5 straight and protruding ribs, particularly developed on the commissure. It has a strongly spicy smell and a spicy-aromatic, slightly spicy flavour. The mother plant of the fennel fruit is *Foeniculum vulgare,* belonging to the *Umbrelliferae.* The fruit of the caraway, or also Carvi fructus, is made up of the schizocarps of the original diachenes. They are 3-6 mm long, about 1 mm thick, gray-brown, mostly slightly sickle-curved, pointed at both ends and glabrous. On the slightly convex back there are three straight, thin, protruding and clear ribs; on the commissural face, slightly arched at the margin, there are two. The styles with their round stylopodium are often still present on the upper end. It has an aromatic smell and a spicy-aromatic taste. The mother plant of the caraway fruit is the *Carum carvi,* belonging to the *Umbrelliferae.*

The fruit of the cumin consists of straight oval and thread-like achenes, 5-6 mm long, yellow-brown in colour, containing a single seed. The seeds are aromatic and have a characteristic bitter taste and a strong, sweetish odour. The mother plant of the cumin fruit is *Cuminum cyminum* or *Cuminum odorum,* it belongs to the *Apiaceae* (or *Umbralliferae)* family.In one embodiment, said species of fruits comprising seeds or seeds in powder form has a particle size distribution value calculated as Dv50 from 15 $\mu$m to 500 $\mu$m, preferably from 150 $\mu$m to 350 $\mu$m. The Dv50 parameter, like the Dv10 and Dv90 parameters, is an index of the particle size distribution, measured in $\mu$m, and indicates the average volume diameter of the dust particles. The values 10, 50 and 90 indicate respectively that 10, 50 and 90% of the analyzed particles are below the corresponding values in $\mu$m and can be obtained using a laser diffractometer. An example of a laser diffractometer experiment is given in the Examples section of this description.

In one embodiment, said species of fruits comprising seeds or powdered seeds has a specific surface area of from 25 $m^2$/Kg to 600 $m^2$/Kg, preferably from 25 $m^2$/Kg to 70 $m^2$/Kg.

In one embodiment, said fruit species comprising seeds or seeds in powder form has a SPAN value expressed as

$$SPAN = \frac{Dv90 - Dv10}{Dv50}$$ , from 1.5 to 3.

The SPAN is the index that correlates the D90, D10 and D50, through the above formula. A value corresponding to 1 corresponds to a perfect Gaussian distribution, higher values indicate greater poly-dispersion of the distribution.

In one embodiment, said species of fruits comprising seeds or seeds in powder form and said natural gum in powder form are in a respective a weight ratio of 1:1 to 1:8, preferably 1:5 to 1: 7.In order to provide a non-limiting example of an embodiment of the present invention, said fruit species comprising seeds or seeds in powder form, and said natural gum powder are in a weight ratio of 1:7 in formulations which they do not require a granulation step in their preparation, while they are in a ratio by weight of 1:5 in formulations which require a granulation step in their preparation.

Granulation is a technological process in which the particles of a powder (understood as granular matter) are made to adhere to each other to form particles with a larger diameter, called granules. The process is often used in the pharmaceutical field for the preparation of granulates which will be destined for direct use or for subsequent processing for the preparation of other pharmaceutical forms such as capsules or tablets.

In a preferred embodiment, said natural gum is gum arabic and/or gum karaya.Gum arabic is a natural gum also known as acacia gum as it is extracted from two sub-Saharan acacia species: *Acacia senegal* and *Acacia seyal.* Like almost all gums and resins of vegetable origin, it is produced by the plant following a natural "gummy" process which is activated spontaneously to heal a vulnus (wound) to its surface integrity. From a chemical point of view, arabic gum is a mixture of calcium, magnesium and potassium salts of arabic acid. Hydrolysis releases the constituent monosaccharides, namely L-arabinose, D-galactose, L-rhamnose and D-glucuronic acid. At the molecular level, it is composed of glycoproteins and oligosaccharides. The field of use is vast, because it is edible, adhesive, prevents the crystallization of sugars and emulsifies fats. The main uses are the food industry as a natural additive, the pharmaceutical industry as an excipient and component, cosmetics for its filming and fixing properties, and glues and paints as a binder for tempera. Karaya gum is the gummy secretion that comes out of the trunk and branches of some species of the genus *Sterculia* (in particular *Sterculia urens),* spontaneously, or following incisions. These plants are native to the highlands of India and Pakistan, although some gum-producing species also grow in Africa. The karaya gum obtained from *Sterculia scaphigera* (Vietnamese species) is instead extracted from the fruit by maceration.

From a chemical point of view, karaya gum is a complex polysaccharide with a high molecular weight, consisting of the sugars galactose, rhamnose and glucoronic acid. Poorly soluble in water, when it comes into contact with it it swells rapidly, reaching a much higher volume than the initial one. This property explains its various applications in the industrial and health fields. Widely used as a thickener/stabilizer by the food (E416) and cosmetic industries, as well as a bonding agent, and as a bulk laxative gum in the treatment of constipation. For this purpose it can also be associated with sulphate or magnesium oxide.In a further embodiment, said natural gum powder has Dv50 in a range of 60 $\mu$m to 70 $\mu$m, a specific surface area in a range of 100 $m^2$/Kg to 150 $m^2$/Kg, preferably 137 $m^2$/Kg, and a SPAN value in a range from 1 to 1.8, preferably 1.75.

In an embodiment according to any of the embodiments described herein, the mixture object of the present invention consists of:

- powdered caraway fruits comprising seeds and powdered gum arabic in a weight ratio ranging from 1:1 to 1:8, preferably from 1:5 to 1:7; and/or
- powdered fennel fruits comprising seeds and powdered gum arabic in a weight ratio ranging from 1:1 to 1:8, preferably from 1:5 to 1:7; and/or
- powdered cumin fruits comprising seeds and powdered gum arabic in a weight ratio ranging from 1:1 to 1:8, preferably from 1:5 to 1:7.

In a preferred embodiment, said mixture comprises caraway fennel and cumin fruits comprising seeds, in a respective ratio of 1:1:1, and gum arabic powder in a weight ratio ranging from 1:1 to 1:8 , preferably from 1:5 to 1:7.

The same applies when powders of the aforementioned seeds are used. The object of the invention is therefore also a lubricating agent for tabletting, consisting of the mixture of the invention as defined in the present description and in the claims.

The characterization of the basic properties of the powders used in the present invention has shown significantly different values between the fruit powders comprising seeds and the magnesium stearate, both in terms of specific surface area and Dv50, but at the same time the characterization of the indirect properties, such as the index of flowability (I.C) and compressibility (I.H.) showed values comparable to, if not lower than, the indexes obtained for magnesium stearate.

A further object of the present invention is represented by a composition comprising the mixture defined according to the previously described embodiments wherein said composition is a tablet, a capsule or a solid composition and wherein said mixture is the sole lubricating agent comprised in said composition. A non-limiting example of tablets according to any embodiment of the present invention is represented by

simple or coated tablets (sugar coated pills): to be swallowed as such;

tablets: not to be swallowed as such, but dissolved in the mouth;

chewable tablets: to be chewed before swallowing;

sublingual tablets: absorbed by the lingual mucosa (e.g. nitroglycerin);

soluble tablets: to be dissolved in water, they are often effervescent to favour dissolution or improve the flavour;

sterile tablets for preparing injection solutions;

sterile compresses for subcutaneous implants;

tablets to be used as vaginal pessaries, which have almost replaced gelatin pessaries;

tablets for preparing solutions for external or diagnostic use (pregnancy tests, denture sterilizers, etc.);

dental cones. Furthermore, the tablets according to the invention can be coated tablets and said coating can also be a coating for their controlled release.

In one embodiment, said mixture of powdered fruits comprising seeds or of powdered seeds is present in said composition in a concentration of 1% to 35% w/w, preferably 3% to 4.5% in said mixture without natural gum, and preferably from 12 to 33% in said mixture comprising natural gum.

According to one embodiment, said composition is a pharmaceutical, cosmetic, nutraceutical composition, a medical device, a food supplement, food. In the embodiment in which said composition is a pharmaceutical composition, the composition comprises at least one natural active complex system (such as for example one or more plant extracts or fractions thereof) or one or more pharmacologically or physiologically active ingredients, and other powdered natural raw materials in pharmaceutical grade or one or more pharmaceutically acceptable excipients and/or carriers, preferably of natural origin (for example plant extracts or derivatives).Said excipients and/or pharmaceutically acceptable carriers are known to those skilled in the art. A medical device, in the present invention, corresponds to a medical device according to any of the classes described in regulation 745/2017 in EEC Directive 93/42 on medical devices (which also includes substances and not just "devices" in the mechanical sense of the term).

The composition, as mentioned above, may also be in the form of a food, such as a food for special medical purposes, a food supplement or in any form according to the regulatory provisions of the country in which this composition will be produced.

In one embodiment, said composition is in the form of capsules, tablets, powder, granules, capsules, solid compositions (for example solid toothpaste).The use of the mixture as defined by the embodiments of the present description is also an object of the present invention, as a lubricating agent in the preparation of tablets, capsules, powder, granules, capsules, solid compositions (for example solid toothpaste).

A further object of the present invention is the use of natural gum, preferably gum arabic or karava, as a lubricating agent in the preparation of tablets, capsules, solid compositions.

In one embodiment, said natural gum is present in a range of 1% to 35% by weight of said tablet, capsule or solid composition.

In a further embodiment, said natural rubber powder has a Dv50 value in a range of 60 $\mu$m to 70 $\mu$m, a specific surface area in a range of 100 $m^2$/Kg to 150 $m^2$/Kg, preferably 137 $m^2$/Kg, and a SPAN value in a range from 1 to 1.8, preferably 1.75.Summarising, the invention also relates to a drug, cosmetic, nutraceutical, medical device, food supplement, food, consisting of the capsule, tablet or solid composition containing, as sole lubricating agent, the lubricating system of the invention according to any of the embodiments herein described.

In one embodiment, said mixture is comprised within the product in the form of tablets, capsules, solid composition, such as for example solid toothpaste, and is therefore used as an internal lubricant only. However, it can also be used, again as sole lubricant only, for the lubrication of machinery suitable for the preparation of said product forms, and can therefore be used as an external lubricant. The use of the mixture object of the invention for the lubrication of machinery can be carried out by spraying through a nozzle. Therefore, for the purposes of the present invention, for the aforementioned use, a suitable micronization of the powders can be carried out so as to obtain the granulometry suitable for the required use.Therefore, the object of the present invention is also a process for manufacturing capsules or tablets or solid compositions comprising the step of admixing the mixture as defined in the present description and in the claims with a suitable formulation preparation (i.e. with the other substances or ingredients which must be present in said capsules, tablets or compositions), in which the sole lubricating agent is said mixture.The present invention also relates to a process for manufacturing capsules or tablets or solid compositions comprising the following steps:

i) preparing one or more fruit comprising seeds or seed species in the form of powder and/or natural gum, wherein the fruits comprising seeds or the seeds in powder from each of said species contain $C_{16}$-$C_{18}$ fatty acids in a percentage higher than 45% w/w with respect to total fatty acids and a percentage of fatty acids of at least 21% w/w, wherein said species of fruits comprising seeds or seeds in the form of powder are cumin, caraway and fennel and wherein said cumin, caraway and fennel have a weight ratio from 1:1:1 to 1:1:3, preferably 1:1:1;

ii) mixing said one or more species of fruit comprising seeds or seeds in the form of powder and/or said natural gum with a suitable formulation preparation for a time of from 2 to 30 minutes, preferably from 5 to 15 minutes, even more preferably 5 minutes;

iii) subjecting the product obtained in step ii) to compression or encapsulation wherein the components used in steps i) and ii) constitute the sole lubricating agent used in said process.

Optionally, the above described process may comprise, between steps i) and ii) above, a further step of granulating said one or more fruit comprising seeds or seed species in the form of powder and/or said natural gum

The invention also relates to a process for manufacturing capsules or tablets or solid compositions as described above, wherein step ii) is substituted by the following steps:

iia) granulating a formulation preparation with a natural gum;

iib) mixing the granulate obtained in step ii) with said one or more fruit comprising seeds or seed species in the form of powder prepared in step i) until a homogeneous mixture is obtained, for a time from 2 to 30 minutes, preferably from 5 to 15 minutes, even more preferably 5 minutesin one embodiment, said granulate obtained in step iia) is dried until a relative humidity of between 23% and 26% is reached, before mixing in point iib).The steps of granulation, compression or encapsulation, and mixing of the process object of the invention are carried out according to the techniques known in the prior art.

The formulation preparate according to the present description is any preparate for the preparation of tablets, capsules or solid compositions as described anywhere in the present description, to which no lubricating agent has been added. In other words, it corresponds to the preparate to be subjected to the manufacturing process of capsules or tablets or solid products to which the skilled person would commonly add magnesium stearate as a lubricant, or other lubricants commonly used in the pharmaceutical, nutraceutical, cosmetic, food, for the preparation of products that must be compressed or encapsulated by industrial machinery commonly used for this purpose. Therefore, the skilled is able to understand the generic definition of formulation preparate/preparation as the skilled person knows which preparations are suitable for being transformed into tablets, capsules or solid products after the appropriate addition of a lubricating agent.

Examples of such preparations are for example mixtures of powders and/or granules, which can comprise, in addition to pharmaceutical substances such as one or more active ingredients, nutraceuticals, food, cosmetics, also one or more of

diluents: which increase the mass of the tablet allowing the achievement of an adequate volume suitable to be processed;

adsorbents: which are used to aggregate the granules. Furthermore, solvents which, by evaporating, exert an adhesive action (water or alcohol) or otherwise syrupy solutions or sticky substances (pectin, methylcellulose, carboxymethylcellulose, gelatin, etc.) are used;

glidants: these agents interpose themselves between the particles of the powder or granulate to which they are added so as to fill the irregular cavities present on their surface; in this way, the shape of the powders or granulates becomes more regular and friction is reduced;disaggregants: thy decrease the time of disaggregation of the tablets.

Instead of commonly used lubricants, such as for example Ca, Mg, Al stearates, stearyl alcohol, stearic alcohol, cetyl alcohol, palmitic acid, alcohol, starch, PEG, talc, in the process of the present invention only the systems with emergent lubricating properties herein described in the form of mixtures or natural gums are used.

Granulation is a technique that involves increasing particle size by agglomeration, is one of the most significant unitary operations in the production of pharmaceutical forms such as tablets and capsules. During the granulation process, small fine or coarse particles are transformed into large agglomerates called granules.

This technological process can take place following two methods called dry granulation and wet granulation. In both processes, the formation of granules is obtained by establishing bonds between the powder particles. Dry granulation uses mechanical compression (slugs) or compaction (roller compaction) to facilitate agglomeration of the powder particles, while wet granulation involves the use of a liquid granulation (binder/solvent) to promote agglomeration through the formation of a wet mass by adhesion.

To the classic granulation techniques, which involve the use of cylinder compactors, or section kneaders and with zeta or sigma arms or even oscillating and rotary granulators, other more recent ones are added which are listed below: pneumatic granulation granulation, reverse wet granulation, steam granulation, moisture activated dry granulation, thermal bond granulation, melt granulation, frozen granulation, foam granulation. Furthermore, for wet granulation, the granulators employed play an important role in granule formation during the process and they can be classified into three types: low speed, high speed and fluidized bed. Low-speed granulators can be kneaders equipped with arms or oscillating or rotary granulators. High-speed granulators, on the other hand, essentially consist of a cylindrical container, hermetically closed by a lid, on the bottom of which there is a rotating blade (impeller). A crusher (chopper) is inserted inside the container, consisting of a rotating blade, which has the task of breaking up agglomerates that are too voluminous, and a sprayer/nebulizer for adding the binding solution, which can simply be water or a ad hoc solution. Fluidized bed granulation is carried out using an equipment which mixes, wets and dries a bed of powder fluidified by air in a single process. In this device, a flow of air is made to pass from bottom to top through the mass of powder to be granulated, at a speed such as to keep it in turbulent suspension, defined as "fluidized". The binding solution is directly sprayed in counter-current on the mass kept in suspension, in such a way as to determine the agglomeration of the particles and therefore the formation of granules. At a certain point in the process, the air is heated to a desired temperature, so this type of device also carries out the drying process of the solid mass in a single step. The drying is in fact at very low temperatures, due to the fact that the suspended material has a very large surface to be dried.In a preferred embodiment, the lubricating agent of the invention, i.e. said mixture of one or more species of fruits comprising seeds or seeds in the form of powder and/or natural gum as disclosed in the present description and as defined in the claims, is used in such quantity in the processes described above, so as to be present in an overall concentration of from 1% to 35% by weight with respect to said capsule, tablet or solid composition.

As stated above, according to the present invention, said lubricating agent is the sole lubricating agent comprised in said capsule, tablet or solid composition.

It is obviously also an object of the invention, a capsule, tablet or solid composition obtainable by the process according to any of the embodiments described herein.It is evident from the experimental data reported in the figures and described in the Examples section, that the presence of a granulation step allows to reduce the quantity of lubricating mixture in the formulation, passing the fennel-gum system in a ratio of 1:7 at a concentration of 32.34%, to a granulated fennel-gum system in a ratio of 1:5 at a concentration of 12%.

The term comprising may be replaced by the term "consisting of" in any part of the present description and of the claims.

Some examples are provided below which have the purpose of better illustrating the methodologies disclosed in the present description.

## EXAMPLES

The powders used in the examples provided below were characterized in terms of nutritional properties, direct and indirect properties and compared with magnesium stearate.

Below is the composition of the powders in terms of fat content, known to have lubricating properties:

Experiment 1:

To investigate the presence of fatty acids with lubricating properties, samples of magnesium stearate, the powder of fennel, cumin and caraway fruits comprising were sent to an external laboratory for complete nutritional composition analysis. In particular, the GC/FID method was used for the analysis of fatty acids. The analysis of the % of residual water was obtained by gravimetric analysis. The results show that the fatty acids contained in the powders of fruit comprising seeds differ from those present in magnesium stearate, but the predominant fatty acids have the same chain length.

Table 1: Reference nutritional analysis of fruits comprising seeds used compared to magnesium stearate

|  | % of residual water | (%) content of total fatty acids | PREDOMINANT FATTY ACIDS(%) |
|---|---|---|---|
| Magnesium stearate | 2.6 | 90.0 | C16-C18 |

(continued)

|  | % of residual water | (%) content of total fatty acids | PREDOMINANT FATTY ACIDS(%) |
|---|---|---|---|
| CUMIN SEEDS LOOSE IMPALPABLE POWDER | 8.3 | 24.1 | Oleic acid (49.83) |
| CARAWAY SEEDS LOOSE IMPALPABLE POWDER | 7.1 | 23.0 | Oleic acid (41.,01); Linoleic acid(33.90) |
| FENNEL SEEDS LOOSE IMPALPABLE POWDER | 6,3 | 21,4 | Oleic acid (75.08) |

In order to carry out evaluations on the concentration of powder of fruits comprising seeds to be inserted in the mixture for the compression tests, the % of the $C_{16}$-$C_{18}$ chain fatty acids were compared.

Considering the amount of fatty acids contained in 1% magnesium stearate, it is estimated to use variable percentages between 1-5% of powders of fruits comprising seeds.

Experiment 2:

The fruit powders comprising seeds were characterized in terms of bulk and tapped density and of relative humidity %. The density values obtained were used to calculate the relative flowability and compressibility indexes.

Table 2: Characterization of indirect properties.

|  | Rest angle | I.C. | I.H. | Bulk D. | Tapped D. | % REL HUMIDITY |
|---|---|---|---|---|---|---|
| Magnesium stearate | 1.55 | 16.42 | 1.2 | 0.35 | 0.448 | 2.63 |
| CUMIN SEEDS LOOSE IMPALPABLE POWDER | 29.41 | 11.86 | 1.13 | 0.43 | 0.489 | 8.34 |
| CARAWAY SEEDS LOOSE IMPALPABLE POWDER | 35.15 | 11.28 | 1.13 | 0.38 | 0.427 | 7.08 |
| FENNEL SEEDS LOOSE IMPALPABLE POWDER | 30.96 | 10.42 | 1.12 | 0.35 | 0.391 | 6.26 |

From the analysis of the data it emerges that the powders as such are characterized by a flowability index (I.C.) and compressibility (I.H.) comparable, if not lower, to the indexes obtained for magnesium stearate.

Experiment 3:

The particle volume distribution of the powders was determined, in solid form, with a Mastersizer 3000 laser diffractometer, Malvern, UK. About 5-7 g of powder was analyzed in dry form by adopting the following parameters, Background measurement duration: 10 s, Sample measurement duration: 19.56 s, Obscuration low limit: 0.10%, Obscuration high limit: 15%, Air pressure: 2 bar, Hopper gap: 2.50mm.

The parameters used were maintained for all analyzes in order to compare the outputs obtained, automatically calculated using Mastersizer software v3.81:

- The median diameter volume Dv 50 is the diameter at which 50% of the distribution is above and 50% is below (percentile diameter relative to 50%)

- Two average particle size determinations should not differ more than 5% relative and the curve of the two determinations should be the same.

- Dv90, 90% of the volume of distribution remains below this value.

- Dv 10, 10% of the volume of distribution remains below this value.

- Span is the breadth of distribution based on the 10%, 50% and 90% quantile. fruits comprising seeds:

From the comparison of the data, the powders tested have values of specific surface area and average particle distribution which differ from those of magnesium stearate.

Table 3: Characterization of fundamental properties.

|  | Specific surface area [m2/kg] | SPAN | DV50 [$\mu$m] |
|---|---|---|---|
| CARAWAY | 53.07 | 2.694 | 181 |
| CUMIN | 31.89 | 2.016 | 282 |
| FENNEL | 57.65 | 2.797 | 197 |
| MAGNESIUM STEARATE | 600 | 2.279 | 15 |

Experiment 4:

*Definition* of a *range of values attributable to expulsion forces which guarantee the detachment of the tablet from the punch.*

Given a mixture of standard powders suitable for the purpose (79% microcrystalline cellulose, 20% calcium carbonate, 1% magnesium stearate), performance tests were carried out at different mixing times.

The ejection force of the tablets was then evaluated.

The mixing times were related to the ejection forces obtained to construct a target curve (Figure 1). Times longer than 30 minutes are considered not useful, as they can lead to overmixing of the mixture, which has a negative impact on the bioavailability of the APS present in the formula. Furthermore, the value of the maximum ejection force beyond which the tablet press would start to give problems of adhesion of the tablet to the punch, which result in the failure to obtain compliant tablets, was considered for the purposes of the present invention to be equal to 0.50 N.

The range of expulsion forces to be considered valid therefore ranges from 0.05 to 0.50 N.Experiment 5:

*Development of mixtures containing single seed* as *lubricant*

Given a standard mixture, compression tests were carried out in which fruit powders comprising seeds of Caraway, Fennel and Cumin taken individually were used in a percentage which contained a quantity of fatty acids equivalent to that present in 1% magnesium stearate in the same formula.

The tablet ejection force values were then analyzed and compared to the parameters obtained by compressing a base mixture containing 1% magnesium stearate and to the maximum experimental ejection force value on other products. All compression tests were performed with the same operating parameters, and are shown in Figure 2.

Looking at the data obtained, it can be deduced that:

- Indirect indices describing rheological behaviour of the powders as such are relevant with respect to the aptitude of a mixture, in which the single raw material is inserted, to be compressed.
- Surprisingly, the particle volume distribution values (dv50) and the specific surface areas seem to be independent of the success of the compression tests when the investigated raw materials are used.

Although in all tests the expulsion force measured is less than 0.5 N, the values obtained tend to the upper limit set in the study. In conclusion, the powders of fruits comprising seeds used individually would seem to exert a lubricating action, but not completely effective, therefore it may be advantageous to study a system that allows to optimize this lubricating action. Experiment 6:

*Development of blends containing a mix of seed and gum*

In the search of functional synergy, gum arabic was examined.

Gum arabic is a 100% natural raw material obtained from the exudate of *Acacia* trees. Its binding and super-disintegrating properties are known in the literature, while its lubricating properties have never been investigated.

The purpose of the tests is to investigate a possible synergy between gum arabic and powders of fruits seeds in terms of lubricating properties.

A mixture consisting of gum arabic and seeds powder in a 7:1 ratio was premixed and inserted into a standard mixture. Figure 3 shows the ejection force values obtained, compared with the blank and with the mixtures containing the single seeds.

Table 4: Ejection forces obtained during compression tests with mixing times of 5 minutes.

Indicated as "control" are the expulsion forces of standard mixture compositions and magnesium stearate according to table 5, at two different mixing times, respectively in column 3 (mixing 5') and 7 (mixing 30') For the experimental tests, the mixing times with optimal results with magnesium stearate were used: 5' min.

| MIXTURE | EJECTION FORCE [N] |
|---|---|
| CARAWAY | 0.417 |
| CUMIN | 0.467 |
| FENNEL | 0.415 |
| CONTROL mixing 5 min | 0.040 |
| GUM ARABIC 1% | 0.26 |
| GUM ARABIC 10% | 0.28 |
| GUM ARABIC 30% | 0.27 |
| CONTROL mixing 30 min | 0.430 |
| mix CUMIN/CARAWAY/FENNEL | 0.13 |
| MIX ARABIC/FENNEL | 0.01 |
| MIX ARABIC/CUMIN | 0.01 |
| MIX ARABIC/CARAWAY | 0.01 |
| MIX ARABIC/ CU-CA-FE | 0.01 |
| MAXIMUM ACCEPTABLE VALUE | 0.50 |

Other components present in the mixture and operating conditions being equal, the systems consisting of gum arabic and powder of fruits comprising seeds combine to produce lower expulsion force values compared to powders of fruits comprising seeds tested individually and the single magnesium stearate.

Table 5: detailed indication of the experiments carried out and of the data obtained with the different combinations.

The table shows the ejection force data obtained with the mixture of standard powders as such (microcrystalline cellulose and calcium carbonate in a 4:1 ratio), or the mixture of standard powders to which magnesium stearate has been added or the various powders of fruits comprising seeds (caraway, fennel, cumin), or arabic gum, or the systems with emerging properties of the invention (mixtures of powders of different fruits comprising seeds or mixtures of arabic gum and powder of one or more fruits comprising seeds) in the form of powder or granulate.It is evident from the table how the mixing of at least three different powders of fruits comprising seeds or at least one powder of a fruit comprising seeds and gum arabic has a synergistic effect providing a 100% natural lubricating system. In particular, the association of gum arabic and powder of at least one fruit comprising seeds.

Pol. Imp. stands for impalpable powder
The granules in the table are those described in experiment 7
TQ stands for as such

Table 5

| microcristalline cellulose | 80 | 79 | 79 | 79 | 79 | 79 | 79 | 76.9975 | 76.8175 | 76.598 | 76.750 | 54.745 | 54.745 | 54.745 | 54.745 |
|---|---|---|---|---|---|---|---|---|---|---|---|---|---|---|---|
| limestone powder or natural calcium carbonate | 20 | 20 | 20 | 20 | 20 | 20 | 20 | 19.3125 | 19.2725 | 19.198 | 19.25 | 11.915 | 11.915 | 11.915 | 11.915 |
| magnesium stearate | | 1 | 1 | 1 | 1 | 1 | 1 | | | | | | | | |
| caraway pol. imp. | | | | | | | | 3.75 | | | 1 | | | 4.21 | 1.3893 |
| cumin pol. imp | | | | | | | | | 3.91 | | 1 | | 4.21 | | 1.3893 |
| fennel pol. imp | | | | | | | | | | 4.21 | 1 | 4.21 | | | 1.3893 |
| arabic gum powder | | | | | | | | | | | | 28.13 | 28.13 | 28.13 | 28.13 |
| TQ, granulate | | | | | | | | | | | | | | | |
| 355 granulate | | | | | | | | | | | | | | | |
| mixing time (min) | | 1 | 5 | 8 | 10 | 15 | 30 | 5 | 5 | 5 | 5 | 5 | 5 | 5 | 5 |
| ejection force | 0.44 | 0.04 | 0.02 | 0.003 | 0.19 | 0.34 | 0.43 | 0.416 | 0.466 | 0.415 | 0.13 | 0.01 | 0.01 | 0.01 | 0.01 |

In the figures, the data shown in columns 3 and 7 above are shown as 5' control and 30' control.

Experiment 7:

Development of mixtures containing a technologically processed system of fruits comprising seeds and gum

A mix of gum arabic and powders of fennel fruit comprising seeds in a 5:1 ratio was granulated with water and sifted through a vibrating sieve.

Granulation, in particular fluid bed granulation, was carried out by means of equipment which mixes, wets and dries a bed of powder fluidified by air in a single process. In this equipment, a flow of air is made to pass from bottom to top through the mass of powder to be granulated, at a speed such as to keep it in turbulent suspension, defined as "fluidized". The binding solution was directly sprayed in counter-current on the mass kept in suspension, in such a way as to determine the agglomeration of the particles and therefore the formation of granules. At a certain point in the process, the air is heated to a desired temperature, so this type of equipment also carries out the drying process of the solid mass in a single step. The drying is in fact at very low temperatures, due to the fact that the suspended material has a very large surface to be dried. The granulate consisting of acacia gum and fennel seeds in a 5:1 ratio obtained using the described technique was added to a standard mixture and then compressed.

The quantity of raw materials to be used and that of binding solution (water) are shown in table 6 and have been calculated on the basis of the volume of the container.

Table 6

| | Batch size$_{(50\%)}$ 12 LITRES |
|---|---|
| total AMOUNT TO BE GRANULATED | 2100 |
| Imalplable powder amount to be granulated | 420 |
| gum arabic amount to be granulated | 1680 |
| RECCOMMENDED AMOUNT OF WATER TO BE SPRAYED | 1365 |

Table 7 below shows the ranges of the experimental parameters used to obtain the granulate in question:

Table 7

| VARIAZIONE | NOZZLE DIAM | velocità di nebulizzazione(g/ml) | SPRAY PRESSURE(pressione aria lubrificante) | Inlet temp | inlet AIR FLOW (m$^3$/hr) | Inlet Air Humidity (g/Kg) | umidità allo scarico | numero giri pompa% | pressione filtro | pressione prodotto | tempo tot | quantita disoluzione totale spruzzata | Temp prod |
|---|---|---|---|---|---|---|---|---|---|---|---|---|---|
| fase di granulazione | 1.2 | 9a11 | 1-0.8 | 66-45 | 65-80 | 6.7-6.5 | 14-13.4 | 20-30 | 264-363 | 694-892 | 1 ora e 40 minuti | 1143 | 40.7- |
| fase di asciugatura | | | | 80-35 | 95-90 | 6.7-6.6 | 16.8-6.7 | | 190-517 | 801-1054 | 60 minuti | | 25.4- |

| VARIATION | Granulation phase | Drying phase |
|---|---|---|
| Nozzle diam | 1.2 | |
| Nebulization speed (g/ml) | 9 to 11 | |
| Spray pressure (pression of lubricating air) | 1-0.8 | |
| Inlet temp | 66-45 | 80-35 |
| Inlet air flow (m³/hr) | 65-80 | 95-90 |
| Inlet air humidity (g/kg) | 6.7-6.5 | 6.7-6.6 |
| Humidity at the exhaust | 14-13.4 | 16.8-6.7 |
| Number of pump revolutions % | 20-30 | |
| Filter pressure | 264-363 | 190-517 |
| Product pressure | 694-892 | 801-1054 |
| Total time | 1 hour and 40 minutes | 60 minutes |
| Total amount of sprayed solution | 1143 | |
| Product temperature | 40.7-24.9 | 25.4-61.3-38.1 |

The granulate obtained was subjected to particle size analysis by means of a Mastersizer 3000 laser diffractometer (see previous experiments), obtaining the results shown in Figure 4. The granulate as such and the majority screened portion (355 $\mu$m) were also characterized from the point of view of specific surface area, SPAN and Dv50, showing no significant differences.

Table 8: Characterization of the fundamental properties of the granulate and its fraction.

| | Specific surface area [m²/kg] | SPAN | $D_v$50 [$\mu$m] |
|---|---|---|---|
| AS SUCH | 35.95 | 1.818 | 207 |
| 355 $\mu$m | 34.93 | 1.612 | 214 |

Both were evaluated in compression tests.

The ejection force values obtained, compared to the values obtained for the blank are shown in Figure 5.
In fact, the same "gum-powder of fruit comprising seeds" system which has different ratios than those of the non-granulated mixtures (7:1) in point 3), allows to obtain tablets without the addition of magnesium stearate either in the formula or externally. Furthermore, the amount of the processed system added to the mixtures is much less than that used in the mixtures shown in Figure 3 (from 32% to 12%).

*Scheme 1. Process for making capsules or tablets with seed and/or gum powders*

| impalpable powder of seed/fruit | Extracts+Powders |
|---|---|
| Gum | Mixing |
| | Lubricated mixture of granulate |
| | commpression/encapsulation |

The powder of fruits comprising seeds, and the powdered natural gum can optionally be granulated beforehand.

*Scheme 2. Process for making capsules or tablets with seed powders and natural gum*

*Scheme 3. Process for making a charcoal tablet with magnesium stearate.*

The charcoal and maltodextrin are granulated with a fluid bed granulator which carries out (as explained above) both the nucleation and the drying steps.

The granulated product is mixed with magnesium stearate and then compressed to form the finished product with some residual moisture, which will be removed by secondary fluid bed drying.

Scheme 4. Process for making a charcoal tablet without the addition of synthetic stearates.

Charcoal, maltodextrin and gum are mixed and granulated with a granulator at low speed for 15 minutes, then they are dried in an oven.

The granulate produced is mixed with fruit powders including fennel seeds and then compressed forming the finished product with a certain residual moisture, which will be removed with a secondary drying in an oven. *Example of preparation* of a *charcoal tablet without adding synthetic stearates:-* Pulverization of fennel seeds;

- Aqueous granulation of 70% charcoal with 10% rubber and 17.6% maltodextrin for 15 minutes;
- Drying of the granulate until reaching a relative humidity between 23% and 26%;
- Mixing the dried granulate with fennel seed powder in a concentration of 2.4% until obtaining a homogeneous mixture, for a time between 5 and 10 minutes;
- Compression;
- Final drying of the tablets.

Table 9. Composition of charcoal tablets with and without the addition of synthetic stearates.

| Material | Old formula | Newformula |
|---|---|---|
| Charcoal | 70 % +/- 5% p/p | 69.72 % +/- 5% p/p |
| Maltodestrine | 29.5 % +/- 5% p/p | 17.51 % +/- 5% p/p |
| Magnesium stearate | 0.5 % p/p | |
| Gum arabic | | 10.38 % p/p |
| Fennel powder of fruits comprising seeds | | 2.39 % p/p |

**Claims**

1. A mixture consisting of one or more fruit comprising seeds or seed species in the form of powder and natural gum powder or of a mixture of at least three different species of seeds in powder, wherein the fruits comprising seeds or the seeds in powder from each of said species contain $C_{16}$-$C_{18}$ fatty acids in a percentage greater than 45% w/w with respect to the total fatty acids and a percentage of fatty acids of at least 21% w/w,
   wherein said species of fruits comprising seeds or seeds in the form of powder are cumin, caraway and fennel and, wherein said fruit comprising seeds or seed powders of cumin, caraway and fennel have a weight ratio from 1:1:1 to 1:1:3, preferably 1:1:1.

2. The mixture according to claim 1, wherein said natural gum is gum arabic and/or karaya gum.

3. The mixture according to anyone of claims 1 or 2, consisting of:

   - caraway fruits comprising seeds or seeds of in the form of powder and gum arabic powder in a weight ratio ranging from 1:1 to 1:8, preferably from 1:5 to 1:7; and/or
   - fennel fruits comprising seeds or seeds in the form of powder and gum arabic powder in a weight ratio ranging from 1:1 to 1:8, preferably from 1:5 to 1:7; and/or
   - cumin fruits comprising seeds or seeds in the form of powder and gum arabic powder in a weight ratio ranging from 1:1 to 1:8, preferably from 1:5 to 1:7, preferably consisting of fruits comprising seeds or seeds powder of caraway, fennel and cumin in a weight ratio from 1:1:1 to 1:1:3, preferably 1:1:1, and powdered gum arabic in a weight ratio ranging from 1:1 to 1:8, preferably from 1:5 to 1:7.

4. The mixture according to anyone of claims 1 to 3, wherein said powder of fruit comprising seeds or seeds has a granulometric distribution value calculated as Dv50 from 15 $\mu$m to 500 $\mu$m, preferably from 150 $\mu$m to 350 $\mu$m and/or wherein said powder of fruit comprising seeds or seeds has a specific surface area from 25 m$^2$/kg to 600 m$^2$/kg, preferably from 25 m$^2$/kg to 70 m$^2$/Kg and/or wherein said powder of fruit comprising seeds or seeds has a SPAN value expressed as $SPAN = \frac{Dv90 - Dv10}{Dv50}$, from 1.5 to 3, preferably wherein said natural gum powder has a value of Dv50 in a range from 60 $\mu$m to 70 $\mu$m, a specific surface area in a range from 100 m$^2$/Kg to 150 m$^2$/Kg, preferably 137 m$^2$/kg, and a SPAN value in a range from 1 to 1.8, preferably 1.75.

5. A tableting lubricating agent consisting of the mixture according to any one of claims 1 to 4.

6. A capsule, tablet or solid composition comprising the mixture according to anyone of claims from 1 to 4 or the lubricating agent according to claim 5 as sole lubricating agent.

7. The capsule, tablet or solid composition according to claim 6, wherein said mixture according to claim 1 to 4 or said lubricating agent according to claim 5 is present in a concentration of 1% to 35% w/w, preferably 3% to 4.5%, preferably wherein said capsule, tablet or composition consists of ingredients having 100% natural origin.

8. A pharmaceutical, cosmetic, nutraceutical, medical device, food supplement, food, consisting of the capsule, tablet or solid composition as defined in anyone one of claims 6 to 7.

9. Use of the mixture according to anyone of claims 1 to 4, as a lubricating agent in the preparation of tablets, capsules, solid compositions, preferably, wherein said mixture is the sole lubricating agent comprised in the product in the form of a tablet, capsule or solid composition; and/or used for the lubrication of machinery suitable for the preparation of said product forms.

10. A process for the manufacturing of capsules or tablets or solid compositions, comprising the step of admixing the mixture as defined in claims 1 to 4 with a suitable formulation preparation, wherein the sole lubricating agent used in said process is said mixture.

11. A process for manufacturing capsules or tablets or solid compositions comprising the following steps:

    i) preparing one or more fruit comprising seeds or seed species in the form of powder and/or natural gum, wherein the fruits comprising seeds or the seeds in powder from each of said species contain $C_{16}$-$C_{18}$ fatty acids in a percentage higher than 45% w/w with respect to total fatty acids and a percentage of fatty acids of at least 21% w/w, wherein said species of fruits comprising seeds or seeds in the form of powder are cumin, caraway and fennel and wherein said cumin, caraway and fennel have a weight ratio from 1:1:1 to 1:1:3, preferably 1:1:1

ii) mixing said one or more species of cumin, caraway and/or fennel fruit comprising seeds or seeds in the form of powder and said natural gum with a suitable formulation preparation for a time of from 2 to 30 minutes, preferably from 5 to 15 minutes, even more preferably 5 minutes;

iii) subjecting the product obtained in step ii) to compression or encapsulation

wherein the components used in steps i) and ii) constitute the sole lubricating agent used in said process.

12. The process according to claim 11, comprising, between steps i) and ii), a further step of granulating said cumin, caraway and/or fennel fruit comprising seeds or seed in the form of powder and/or said natural gum, alternatively wherein step ii) is substituted by the following steps:

iia) granulating a formulation preparation with a natural gum;

iib) mixing the granulate obtained in step ii) with said one or more cumin, caraway and/or fennel fruit comprising seeds or seed in the form of powder prepared in step i) until a homogeneous mixture is obtained, for a time from 2 to 30 minutes, preferably from 5 to 15 minutes, even more preferably 5 minutes, preferably wherein said granulate obtained in step iia) is dried until a relative humidity of between 23% and 26% is reached, before mixing in point iib), more preferably wherein said granulation is a dry granulation, a wet granulation, a dry pneumatic granulation, an inverse wet granulation, a steam granulation, an activated-from-moisture dry granulation, a thermal adhesion granulation, a melt granulation, a freeze granulation or foam granulation.

13. The process according to anyone one of claims 10 to 12, wherein said cumin, caraway and/or fennel fruit comprising seeds or seed species in the form of powder and natural gum are present in an overall concentration from 1% to 35% by weight with respect to said capsule, tablet or solid composition.

14. The process according to anyone one of claims 12 to 13 wherein said natural gum is gum arabic and/or karaya gum.

15. A capsule, tablet or solid composition obtainable by the process according to anyone one of claims 10 to 14.

**Patentansprüche**

1. Mischung bestehend aus einer oder mehreren Früchten, umfassend Samen oder Samenarten in der Form von Pulver, und natürlichem Gummipulver oder einer Mischung aus mindestens drei unterschiedlichen Samenarten in Pulver, wobei die Früchte umfassend Samen oder die Samen in Pulver von jeder der Arten $C_{16}$-$C_{18}$-Fettsäuren in einem Prozentsatz von über 45 Gew.-% hinsichtlich der gesamten Fettsäuren und einen Prozentsatz an Fettsäuren von mindestens 21 Gew.-% enthalten, wobei die Arten von Früchten, umfassend Samen oder Samen in der Form von Pulver, Kreuzkümmel, Kümmel und Fenchel sind, und wobei die Früchte, umfassend Samen oder Samenpulver von Kreuzkümmel, Kümmel und Fenchel, ein Gewichtsverhältnis von 1 : 1 : 1 bis 1 : 1 : 3, vorzugsweise 1 : 1 : 1, aufweisen.

2. Mischung nach Anspruch 1, wobei der natürliche Gummi Gummi arabicum und/oder Karayagummi ist.

3. Mischung nach einem der Ansprüche 1 oder 2 bestehend aus:

- Kümmelfrüchten, umfassend Samen oder Samen von in der Form von Pulver, und Gummi arabicum-Pulver in einem Gewichtsverhältnis, das von 1 : 1 bis 1 : 8, vorzugsweise von 1 : 5 bis 1 : 7, reicht; und/oder
- Fenchelfrüchten, umfassend Samen oder Samen in der Form von Pulver, und Gummi arabicum-Pulver in einem Gewichtsverhältnis, das von 1 : 1 bis 1 : 8, vorzugsweise von 1 : 5 bis 1 : 7, reicht; und/oder
- Kreuzkümmelfrüchten, umfassend Samen oder Samen in der Form von Pulver, und Gummi arabicum-Pulver in einem Gewichtsverhältnis, das von 1 : 1 bis 1 : 8, vorzugsweise von 1 : 5 bis 1 : 7, reicht, vorzugsweise bestehend aus Früchten, umfassend Samen oder Samenpulver von Kümmel, Fenchel und Kreuzkümmel in einem Gewichtsverhältnis von 1 : 1 : 1 bis 1 : 1 : 3, vorzugsweise 1 : 1 : 1, und Gummi arabicum-Pulver in einem Gewichtsverhältnis, das von 1 : 1 bis 1 : 8, vorzugsweise von 1 : 5 bis 1 : 7, reicht.

4. Mischung nach einem der Ansprüche 1 bis 3, wobei das Pulver von Frucht, umfassend Samen oder Samen, einen Korngrößenverteilungswert, der als Dv50 berechnet wird, von 15 $\mu$m bis 500 $\mu$m, vorzugsweise von 150 $\mu$m bis 350

µm, aufweist, und/oder wobei das Pulver von Frucht, umfassend Samen oder Samen, eine spezifische Oberfläche von 25 m²/kg bis 600 m²/kg, vorzugsweise von 25 m²/kg bis 70 m²/kg, aufweist, und/oder wobei das Pulver von Frucht, umfassend Samen oder Samen, einen SPAN-Wert, der als $SPAN = \frac{Dv90 - Dv10}{Dv50}$, von 1,5 bis 3 ausgedrückt wird, aufweist, vorzugsweise wobei das natürliche Gummipulver einen Dv50-Wert in einem Bereich von 60 µm bis 70 µm, eine spezifische Oberfläche in einem Bereich von 100 m²/kg bis 150 m²/kg, vorzugsweise 137 m²/kg, und einen SPAN-Wert in einem Bereich von 1 bis 1,8, vorzugsweise 1,75, aufweist.

5. Tablettierschmiermittel bestehend aus der Mischung nach einem der Ansprüche 1 bis 4.

6. Kapsel, Tablette oder feste Zusammensetzung, umfassend die Mischung nach einem der Ansprüche 1 bis 4 oder das Schmiermittel nach Anspruch 5 als einziges Schmiermittel.

7. Kapsel, Tablette oder feste Zusammensetzung nach Anspruch 6, wobei die Mischung nach Anspruch 1 bis 4 oder das Schmiermittel nach Anspruch 5 in einer Konzentration von zu 1 Gew.-% bis 35 Gew.-%, vorzugsweise 3 Gew.-% bis 4,5 Gew.-%, vorliegt, vorzugsweise wobei die Kapsel, die Tablette oder die Zusammensetzung aus Inhaltsstoffen, die zu 100 % natürlichen Ursprungs sind, besteht.

8. Arzneimittel, Kosmetikum, Nutrazeutikum, Medizinprodukt, Nahrungsergänzungsmittel, Lebensmittel bestehend aus der Kapsel, der Tablette oder der festen Zusammensetzung wie in einem der Ansprüche 6 bis 7 definiert.

9. Verwendung der Mischung nach einem der Ansprüche 1 bis 4 als ein Schmiermittel bei der Herstellung von Tabletten, Kapseln, festen Zusammensetzungen, vorzugsweise wobei die Mischung das einzige Schmiermittel ist, das in dem Produkt in der Form einer Tablette, Kapsel oder festen Zusammensetzung enthalten ist; und/oder Verwendung für die Schmierung von Maschinen, die für die Herstellung der Produktformen geeignet sind.

10. Verfahren für die Fertigung von Kapseln oder Tabletten oder festen Zusammensetzungen, umfassend den Schritt eines Zumischens der Mischung, wie in den Ansprüchen 1 bis 4 definiert, mit einer geeigneten Formulierungsherstellung, wobei das einzige in dem Verfahren verwendete Schmiermittel die Mischung ist.

11. Verfahren zum Fertigen von Kapseln oder Tabletten oder festen Zusammensetzungen, umfassend die folgenden Schritte:

i) Herstellen einer oder mehrerer Früchte, umfassend Samen oder Samenarten in der Form von Pulver, und/oder von natürlichem Gummi, wobei die Früchte, umfassend Samen oder die Samen in Pulver von jeder der Arten $C_{16}$-$C_{18}$-Fettsäuren in einem Prozentsatz von höher als 45 Gew.-% hinsichtlich der Gesamtfettsäuren und einem Prozentsatz an Fettsäuren von mindestens 21 Gew.-% enthalten, wobei die Arten von Früchten, umfassend Samen oder Samen in der Form von Pulver Kreuzkümmel, Kümmel und Fenchel sind, und wobei der Kreuzkümmel, Kümmel und Fenchel ein Gewichtsverhältnis von 1 : 1 : 1 bis 1 : 1 : 3, vorzugsweise 1 : 1 : 1, aufweisen.
ii) Mischen der einen oder der mehreren Arten von Kreuzkümmel-, Kümmel- und/oder Fenchelfrucht, umfassend Samen oder Samen in der Form von Pulver, und des natürlichen Gummis mit einer geeigneten Herstellung für eine Zeit von 2 bis 30 Minuten, vorzugsweise von 5 bis 15 Minuten, noch mehr bevorzugt von 5 Minuten;
iii) Unterziehen des Produkts, das in Schritt ii) erhalten wird, einer Kompression oder Verkapselung, wobei die Komponenten, die in den Schritten i) und ii) verwendet werden, das einzige in dem Verfahren verwendete Schmiermittel darstellen.

12. Verfahren nach Anspruch 11, umfassend, zwischen den Schritten i) und ii) einen weiteren Schritt eines Granulierens der Kreuzkümmel-, Kümmel- und/oder Fenchelfrucht, umfassend Samen oder Samen in Form von Pulver, und/oder des natürlichen Gummis, alternativ wobei Schritt ii) durch die folgenden Schritte ersetzt wird:

iia) Granulieren einer Formulierungsherstellung mit einem natürlichen Gummi;
iib) Mischen des in Schritt ii) erhaltenen Granulats mit dem einen oder den mehreren in Schritt i) hergestellten von Kreuzkümmel-, Kümmel-, und/oder Fenchelfrucht, umfassend Samen, oder Samen in der Form von Pulver, bis eine homogene Mischung erhalten wird, für eine Zeit von 2 bis 30 Minuten, vorzugsweise von 5 bis 15 Minuten, noch mehr bevorzugt von 5 Minuten, vorzugsweise wobei das in Schritt iia) erhaltene Granulat getrocknet wird, bis eine relative Luftfeuchtigkeit zwischen 23 % und 26 % erreicht ist, bevor es in Punkt iib) vermischt wird, mehr bevorzugt wobei die Granulierung eine Trockengranulierung, eine Feuchtgranulierung, eine pneumatische Trockengranulierung, eine inverse Feuchtgranulierung, eine Dampfgranulierung, eine durch Feuchtigkeit aktivierte Trockengranulierung, eine thermische Adhäsionsgranulierung, eine Schmelzgranulierung, eine Gefriergranulierung oder eine Schaumgranulierung ist.

**13.** Verfahren nach einem der Ansprüche 10 bis 12, wobei die Kreuzkümmel-, Kümmel-, und/oder Fenchelfrucht, umfassend Samen oder Samenarten, in der Form von Pulver, und der natürliche Gummi in einer Gesamtkonzentration von 1 Gew.-% bis 35 Gew.-% hinsichtlich der Kapsel, der Tablette oder der festen Zusammensetzung vorliegen.

**14.** Verfahren nach einem der Ansprüche 12 bis 13, wobei der natürliche Gummi Gummi arabicum und/oder Karayagummi ist.

**15.** Kapsel, Tablette oder feste Zusammensetzung, die durch das Verfahren nach einem der Ansprüche 10 bis 14 erhältlich ist.

**Revendications**

**1.** Mélange constitué d'une ou plusieurs espèces de fruits comprenant des graines ou de graines sous forme de poudre et de poudre de gomme naturelle ou d'un mélange d'au moins trois espèces différentes de graines en poudre, dans lequel les fruits comprenant des graines ou les graines en poudre de chacune desdites espèces contiennent des acides gras en $C_{16}$-$C_{18}$ dans un pourcentage supérieur à 45 % p/p par rapport aux acides gras totaux et un pourcentage d'acides gras d'au moins 21 % p/p,
dans lequel lesdites espèces de fruits comprenant des graines ou de graines sous forme de poudre sont le cumin, le carvi et le fenouil et, dans lequel lesdites poudres de fruits comprenant des graines ou de graines de cumin, carvi et fenouil ont un rapport en poids allant de 1:1:1 à 1:1:3, de préférence de 1:1:1.

**2.** Mélange selon la revendication 1, dans lequel ladite gomme naturelle est la gomme arabique et/ou la gomme karaya.

**3.** Mélange selon l'une quelconque des revendications 1 ou 2, constitué de :

- fruits comprenant des graines ou graines de carvi sous forme de poudre et poudre de gomme arabique dans un rapport en poids allant de 1:1 à 1:8, de préférence de 1:5 à 1:7 ; et/ou
- fruits comprenant des graines ou graines de fenouil sous forme de poudre et poudre de gomme arabique dans un rapport en poids allant de 1:1 à 1:8, de préférence de 1:5 à 1:7 ; et/ou
- fruits comprenant des graines ou graines de cumin sous forme de poudre et poudre de gomme arabique dans un rapport en poids allant de 1:1 à 1:8, de préférence de 1:5 à 1:7, constitué de préférence de poudre de fruits comprenant des graines ou de graines de carvi, fenouil et cumin dans un rapport en poids allant de 1:1:1 à 1:1:3, de préférence de 1:1:1, et de gomme arabique en poudre dans un rapport en poids allant de 1:1 à 1:8, de préférence de 1:5 à 1:7.

**4.** Mélange selon l'une quelconque des revendications 1 à 3, dans lequel ladite poudre de fruit comprenant des graines ou de graines a une valeur de distribution granulométrique calculée en tant que Dv50 de 15 $\mu$m à 500 $\mu$m, de préférence de 150 $\mu$m à 350 $\mu$m et/ou dans lequel ladite poudre de fruit comprenant des graines ou de graines a une surface spécifique allant de 25 m$^2$/kg à 600 m$^2$/kg, de préférence de 25 m$^2$/kg à 70 m$^2$/kg et/ou dans lequel ladite poudre de fruit comprenant des graines ou de graines a une valeur SPAN exprimée en tant que

$$SPAN = \frac{Dv90 - Dv10}{Dv50},$$ allant de 1,5 à 3, de préférence dans lequel ladite poudre de gomme naturelle a une valeur de Dv50 comprise dans une plage allant de 60 $\mu$m à 70 $\mu$m, une surface spécifique comprise dans une plage allant de 100 m$^2$/kg à 150 m$^2$/kg, de préférence de 137 m$^2$/kg, et une valeur SPAN comprise dans une plage allant de 1 à 1,8, de préférence de 1,75.

**5.** Agent lubrifiant de fabrication de comprimés constitué du mélange selon l'une quelconque des revendications 1 à 4.

**6.** Gélule, comprimé ou composition solide comprenant le mélange selon l'une quelconque des revendications 1 à 4 ou l'agent lubrifiant selon la revendication 5 en tant qu'unique agent lubrifiant.

**7.** Gélule, comprimé ou composition solide selon la revendication 6, dans lequel ledit mélange selon les revendications 1 à 4 ou ledit agent lubrifiant selon la revendication 5 est présent en une concentration de 1 % à 35 % p/p, de préférence de 3 % à 4,5 %, de préférence dans lequel ladite gélule, ledit comprimé ou ladite composition est constitué d'ingrédients d'origine 100 % naturelle.

8. Produit pharmaceutique, produit cosmétique, produit nutraceutique, dispositif médical, complément alimentaire, aliment, constitué d'une gélule, d'un comprimé ou d'une composition solide selon l'une quelconque des revendications 6 à 7.

9. Utilisation du mélange selon l'une quelconque des revendications 1 à 4, en tant qu'agent lubrifiant dans la préparation de comprimés, gélules, compositions solides, de préférence, dans laquelle ledit mélange est l'unique agent lubrifiant compris dans le produit sous forme de comprimé, gélule ou composition solide ; et/ou utilisé pour la lubrification de machines adaptées à la préparation desdites formes de produits.

10. Procédé de fabrication de gélules, comprimés ou compositions solides, comprenant l'étape consistant à mélanger le mélange selon les revendications 1 à 4 avec une préparation de formulation appropriée, dans lequel l'unique agent lubrifiant utilisé dans ledit procédé est ledit mélange.

11. Procédé de fabrication de gélules, comprimés ou compositions solides comprenant les étapes suivantes :

   i) préparation d'une ou plusieurs espèces de fruits comprenant des graines ou de graines sous forme de poudre et/ou de gomme naturelle, dans lequel les fruits comprenant des graines ou les graines en poudre de chacune desdites espèces contiennent des acides gras en $C_{16}$-$C_{18}$ dans un pourcentage supérieur à 45 % p/p par rapport aux acides gras totaux et un pourcentage d'acides gras d'au moins 21 % p/p, dans lequel lesdites espèces de fruits comprenant des graines ou de graines sous forme de poudre sont le cumin, le carvi et le fenouil et dans lequel ledit cumin, ledit carvi et ledit fenouil ont un rapport en poids allant de 1:1:1 à 1:1:3, de préférence de 1:1:1
   ii) mélange de ladite ou desdites espèces de fruits comprenant des graines ou de graines de cumin, carvi et/ou fenouil sous forme de poudre et de ladite gomme naturelle avec une préparation de formulation appropriée pendant une durée allant de 2 à 30 minutes, de préférence de 5 à 15 minutes, encore plus préférablement de 5 minutes ;
   iii) le fait de soumettre le produit obtenu à l'étape ii) à une compression ou une encapsulation, dans lequel les composants utilisés aux étapes i) et ii) constituent l'unique agent lubrifiant utilisé dans ledit procédé.

12. Procédé selon la revendication 11, comprenant, entre les étapes i) et ii), une étape supplémentaire de granulation desdits fruits comprenant des graines ou desdites graines de cumin, carvi et/ou fenouil sous forme de poudre et/ou de ladite gomme naturelle, dans lequel, en variante, l'étape ii) est remplacée par les étapes suivantes :

   iia) granulation d'une préparation de formulation avec une gomme naturelle ;
   iib) mélange du granulat obtenu à l'étape ii) avec ledit ou lesdits fruits comprenant des graines ou ladite ou lesdites graines de cumin, carvi et/ou fenouil sous forme de poudre préparés à l'étape i) jusqu'à l'obtention d'un mélange homogène, pendant une durée allant de 2 à 30 minutes, de préférence de 5 à 15 minutes, encore plus préférablement de 5 minutes, de préférence dans lequel ledit granulat obtenu à l'étape iia) est séché jusqu'à l'obtention d'une humidité relative comprise entre 23 % et 26 %, avant d'être mélangé au point iib), plus préférablement dans lequel ladite granulation est une granulation par voie sèche, une granulation par voie humide, une granulation pneumatique par voie sèche, une granulation par voie humide inverse, une granulation à la vapeur, une granulation par voie sèche activée à partir de l'humidité, une granulation par adhésion thermique, une granulation par fusion, une granulation par congélation ou une granulation par mousse.

13. Procédé selon l'une quelconque des revendications 10 à 12, dans lequel lesdites espèces de fruits comprenant des graines ou de graines de cumin, carvi et/ou fenouil sous forme de poudre et ladite gomme naturelle sont présentes en une concentration globale allant de 1 % à 35 % en poids par rapport à ladite gélule, audit comprimé ou à ladite composition solide.

14. Procédé selon l'une quelconque des revendications 12 à 13, dans lequel ladite gomme naturelle est la gomme arabique et/ou la gomme karaya.

15. Gélule, comprimé ou composition solide pouvant être obtenu par le procédé selon l'une quelconque des revendications 10 à 14.

## EJECTION FORCE [N]

Fig. 1

## EJECTION FORCE [N]

| | |
|---|---|
| CARAWAY | 0.417 |
| CUMIN | 0.467 |
| FENNEL | 0.415 |
| CONTROL 5 MIN | 0.040 |
| CONTROL 30 MIN | 0.430 |
| MAX VALUE | 0.500 |

Fig. 2

## EJECTION FORCE [N]

CARAWAY

CUMIN

FENNEL

CONTROL 6

CONTROL 30

mix CUMIN/CARAWAY/FENNEL

MIX ARABIC/FENNEL 0.01

MIX ARABIC/CUMIN 0.01

MIX ARABIC/CARAWAY 0.01

MIX ARABIC CU/CA/FE 0.01

MAX VALUE

Fig. 3

# GRANULOMETRIC DISPERSION %

0.00    0.06    0.69    4.36    19.98    40.69    25.71    8.70

0    63    125    180    250    355    500    710

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007320856 B
- US 2020170936 A
- KR 20200009128